# FASCICULE DE BREVET EUROPEEN

(11) **EP 2 459 079 B1**
(45) Date de publication et mention de la délivrance du brevet: **07.05.2014**
(21) Numéro de dépôt: 10752867.1
(22) Date de dépôt: 26.07.2010
(51) Int. Cl.: A61B 17/132

(54) **DISPOSITIF HÉMOSTATIQUE PAR COMPRESSION**
KOMPRIMIERBARE BLUTSTILLENDE VORRICHTUNG
COMPRESSIVE HEMOSTATIC DEVICE

(30) Priorité: 31.07.2009 FR 0955412
(43) Date de publication de la demande: 06.06.2012
(73) Titulaire: Perouse Medical, 60173 Ivry le Temple (FR)
(72) Inventeur: GUILLOT, Romain, Christian, F-01390 Mionnay (FR)
(74) Mandataire: Blot, Philippe Robert Emile
(86) Numéro de dépôt international: PCT/FR2010/051577
(87) Numéro de publication internationale: WO 2011/012805

(56) Documents cités:
- DE-U1-202009 005 566
- GB-A- 191 508 900
- US-A- 4 760 846
- US-A1- 2004 122 469
- US-A1- 2008 177 159

## Description

La présente invention concerne un dispositif hémostatique par compression du type décrit dans le préambule de la revendication 1. Ce dispositif est notamment adapté à réaliser l'hémostase de l'artère radiale suite à une intervention, par exemple, d'angiographie ou d'angioplastie sans gêner le retour veineux et sans comprimer l'artère ulna.

Certains procédés d'imagerie médicale nécessitent une intervention médicale consistant à introduire un produit de contraste radiologique dans le système sanguin du patient. L'injection de produit de contraste se fait à travers des cathéters que l'on passe dans un introducteur débouchant dans un vaisseau sanguin, l'introducteur assurant l'étanchéité du montage. Par exemple, l'introducteur est placé sur la face interne du poignet du patient de sorte à permettre l'injection du liquide via l'artère radiale. Une fois l'intervention terminée, le retrait de l'introducteur en limitant les saignements nécessite l'application d'une pression suffisante au niveau du point où la ponction a été réalisée, dans une zone qualifiée de zone d'introduction ou de ponction. La compression est réduite de façon progressive, jusqu'à l'hémostase du vaisseau sanguin concerné ce qui peut prendre plusieurs heures.

Plusieurs dispositifs peuvent être employés pour obtenir une telle pression sur la zone d'introduction. Le document GB 08900 par exemple, divulgue un dispositif hémostatique du type précité. Les praticiens utilisent par ailleurs des dispositifs constitués de pansements enserrant la zone d'introduction. Le dispositif de GB 08900, et ces dispositifs constitués de pansements ne permettent ni un positionnement optimal sur la zone d'introduction, de par leur caractère opaque, ni une évaluation fiable de la pression exercée. Or ces paramètres déterminent de manière fondamentale l'efficacité d'un tel dispositif.

Il existe encore des dispositifs, par exemple le « TR-BAND ® » de la société TERUMO, prenant la forme d'un bracelet translucide comprenant des coussinets gonflables. Le bracelet est positionné autour du poignet du patient sur la zone d'introduction. Une seringue est alors utilisée pour gonfler les coussinets, lesquels exercent une pression sur la zone d'introduction. Ce type de dispositif comporte donc plusieurs éléments, ce qui complexifie les manipulations par le personnel soignant. En outre, ces dispositifs ne permettent pas davantage d'évaluer avec fiabilité et simplicité la pression exercée lors de la pose du dispositif, ni le ou les paliers de décompression.

Le document DE 20 2009 005566 U1 qui forme la base du préambule de la revendication 1, divulgue un dispositif hémostatique qui comprend:
- une embase (6);
- des moyens de maintien (5) aptes à fixer temporairement l'embase au patient;
- un applicateur porté par l'embase et muni d'un tampon (1); et
- des moyens de réglage (8) aptes à entraîner l'applicateur vers la zone d'introduction de sorte que l'applicateur se déplace jusqu'à une position où le tampon (1) exerce une pression sur la zone d'introduction.

L'invention a pour but de proposer un dispositif autonome, pouvant être posé et manipulé par un unique intervenant, à tout moment, de manière simple et fiable.

En outre, afin de gérer au mieux l'hémostase, le personnel soignant doit suivre de manière efficace et fiable la durée de compression effective de la zone d'introduction du patient. En effet, non seulement il est souhaitable de connaître à tout instant la durée totale de compression afin que le personnel soignant puisse évaluer la pertinence du retrait du dispositif hémostatique, mais il est encore recommandé d'effectuer l'étape de décompression par paliers.

Les dispositifs connus jusqu'alors ne disposent d'aucun moyen particulier permettant de mémoriser de manière efficace l'heure de pose. Tout au plus est-il possible d'écrire l'heure de pose avec un simple stylo sur le dispositif lui-même ou sur la zone traitée sur le patient. Aussi les risques d'effacement sont bien réels et le personnel soignant doit calculer sans aide l'heure de retrait, ce qui est susceptible d'entraîner des erreurs de calcul préjudiciables. En outre, le suivi horaire de multiples paliers de décompression s'avère particulièrement difficile, puisque les problèmes précédemment cités se trouvent multipliés par le suivi de plusieurs patients, et la place limitée finit par rendre l'opération trop complexe.

L'invention a encore pour but de proposer un dispositif horaire permettant de suivre de manière simple et fiable la durée totale de pose du dispositif médical, et/ou la durée d'étapes intermédiaires réalisées à l'aide dudit dispositif médical, ce dispositif horaire pouvant notamment être utilisé sur ou avec un dispositif hémostatique tel que mentionné plus haut.

A cet effet, la présente invention a pour objet un dispositif hémostatique par compression du type précité, caractérisé par la partie caractérisante de la revendication 1.

Des modes particuliers de réalisation, sont décrits dans les revendications 2 à 15.

D'autres caractéristiques et avantages de la présente invention apparaîtront mieux à la lecture de la description qui suit d'un exemple de réalisation donné à titre illustratif et nullement limitatif annexé des figures qui représentent :
- la figure 1, une vue d'ensemble en perspective d'un dispositif hémostatique par compression selon un premier mode de réalisation de l'invention ;
- les figures 2a et 2b, une vue frontale et latérale de l'embase du dispositif hémostatique par compression selon le premier mode de réalisation ;
- les figures 3a et 3b, une vue latérale et une coupe axiale de l'applicateur du dispositif hémostatique par compression selon le premier mode de réalisation ;
- les figures 3c et 3d, une vue en plan et une coupe axiale suivant la ligne IIId-IIId de la figure 3c, respectivement, d'une variante d'applicateur ;
- la figure 4a, une coupe latérale du dispositif hémostatique par compression selon le premier mode de réalisation ;
- la figure 4b, une coupe axiale du bouton du dispositif hémostatique par compression selon le premier mode de réalisation ;
- la figure 5, une vue frontale d'un bracelet du premier mode de réalisation ;
- les figures 6a, 6b, 6c, une coupe latérale, une vue latérale et une vue frontale du dispositif hémostatique par compression selon un deuxième mode de réalisation de l'invention;
- les figures 7a, 7b, 7c, deux coupes latérales et une vue frontale des moyens de mémorisation des heures de pose et de retrait selon un premier mode de réalisation ;
- les figures 8a, 8b, une coupe latérale et une vue frontale des moyens de mémorisation des heures de pose et de retrait selon un deuxième mode de réalisation ;
- la figure 9, une vue éclatée du dispositif hémostatique par compression selon un troisième mode de réalisation de l'invention, le tampon transparent n'étant pas représenté ;
- la figure 10, une vue en perspective d'un loquet anti-retour du dispositif de la figure 9 ;
- la figure 11, une vue de dessus du dispositif de la figure 9 ;
- la figure12, une vue en perspective du loquet anti-retour de la figure 10 positionné sur l'embase du dispositif de la figure 9;
- la figure 13, une coupe axiale du bouton du dispositif de la figure 9 avec le loquet anti-retour de la figure 10 en position de blocage ;
- la figure 14, une coupe axiale analogue à celle de la figure 13 mais avec le loquet anti-retour en position de déblocage.

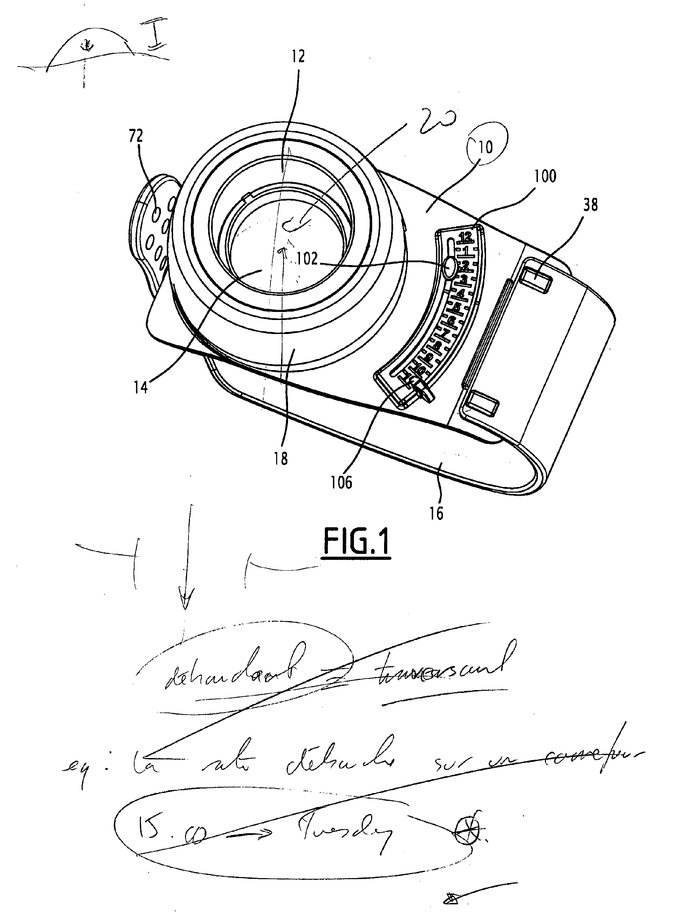

Le dispositif hémostatique par compression selon un premier mode de réalisation illustré sur la figure 1 comporte une embase 10 sur laquelle est monté un applicateur 12 comportant à l'une de ses extrémités un tampon 14. Sur l'embase 10 vient se fixer un bracelet 16 permettant de fixer temporairement l'embase 10 au patient. Le dispositif comporte encore un bouton 18 rotatif monté sur l'embase 10 et coopérant avec l'applicateur 12 de sorte à entraîner en translation ce dernier vers la zone d'introduction ou de ponction et inversement.

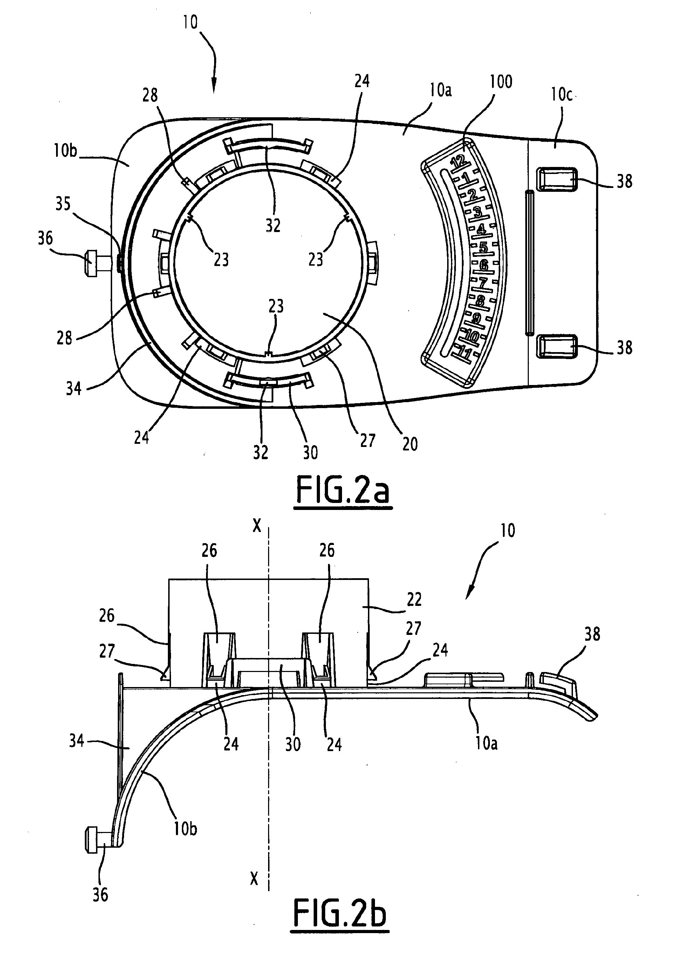

L'embase 10, telle que représentée sur les figures 2a et 2b, est formée d'une partie plane 10a prolongée par une partie incurvée 10b en arc de cercle. La forme générale ainsi que les dimensions de l'embase 10 sont choisies de sorte à ce que l'embase 10 puisse être positionnée de manière ergonomique autour de la zone d'introduction sur un poignet du patient. L'embase 10 est traversée par un évidement sensiblement circulaire formant un trou de visée 20, dont le centre se situe au milieu du segment constituant la frontière entre la partie plane 10a et la partie incurvée 10b.

Le trou de visée 20 forme la base d'un collet 22 sensiblement cylindrique. L'axe X-X du collet 22, supposé vertical pour les besoins de la présente description, est perpendiculaire à la partie plane 10a de l'embase 10. Trois glissières 23 de guidage linéaire s'étendent axialement tout le long de la face interne du collet 22. Les glissières 23 sont équidistantes deux à deux.

En variante, une des glissières peut être légèrement différente ou décalée de façon à indexer l'applicateur sur l'embase.

Des trous débouchants 24 sont formés à la base et autour du collet 22 et se prolongent dans la partie attenante de l'embase 10. Les trous débouchants 24 sont répartis uniformément sur toute la périphérie du collet 22. Le collet 22 se prolonge dans les trous débouchants 24 en clips d'assemblage 26. Les clips d'assemblage 26 s'étendent parallèlement à l'axe X-X. Chaque clip d'assemblage 26 comprend à son extrémité distale une saillie 27 orientée vers l'extérieur du collet 22. Chaque saillie 27 est saillante par rapport à la surface extérieure du collet 22.

L'embase 10 comporte dans sa partie incurvée 10b et en périphérie du collet 22 des nervures radiales 28 de rigidification et d'appui du bouton de réglage. Les nervures 28 s'élèvent jusqu'à hauteur de la partie plane 10a. Les nervures radiales 28 ont leur face supérieure plane comprise dans le même plan que celui de la partie plane 10a de l'embase 10.

L'embase 10 comporte, à cheval sur le segment formant la frontière entre la partie plane 10a et la partie incurvée 10b, deux supports 30 opposés diamétralement par rapport à l'axe X-X. Chaque support 30 s'élève verticalement. Les faces latérales de chaque support 30 forment un arc de cercle centré sur l'axe X-X. Chaque support 30 est déformable élastiquement et comporte sur sa face latérale intérieure un godron ou saillie de cliquet 32.

La partie incurvée 10b de l'embase 10 comporte une extension 34 de forme semi-cylindrique creuse d'axe longitudinal X-X. L'extension 34 s'élève jusqu'à hauteur du plan de la partie plane 10a. L'extension 34 a un diamètre sensiblement égal à la longueur du segment formant la frontière entre la partie plane 10a et la partie incurvée 10b.

L'embase 10 comprend à l'extrémité de sa partie incurvée 10b un plot de fixation 36. L'embase 10 comprend à l'extrémité de sa partie plane 10a un prolongement 10c supportant deux crochets 38.

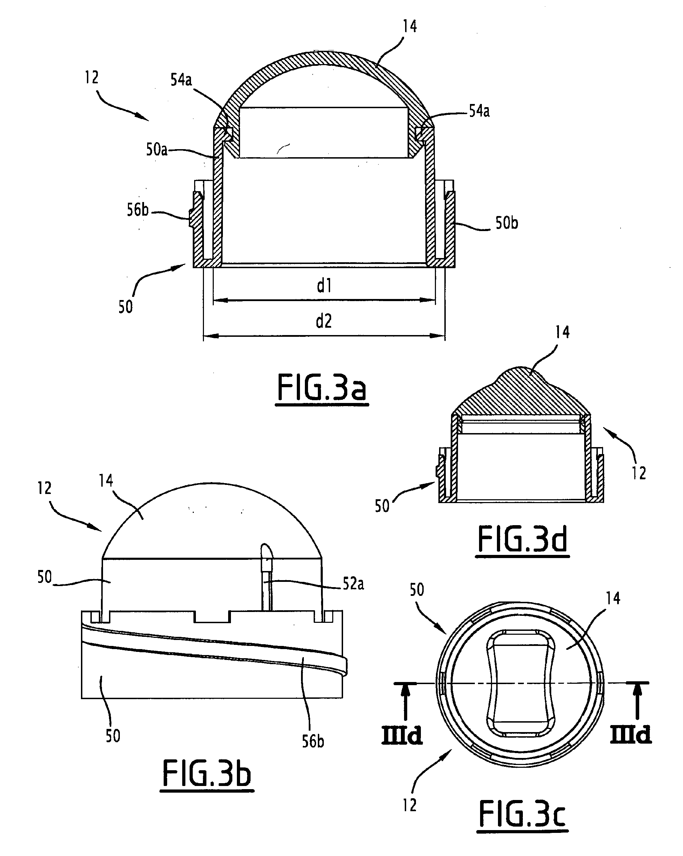

Comme illustré sur les figures 3a et 3b, l'applicateur 12 comporte un support 50. Le support 50 est formé d'un tube intérieur 50a cylindrique de diamètre extérieur d₁ et d'un tube extérieur 50b cylindrique de diamètre intérieur d₂. Les deux tubes 50a et 50b sont coaxiaux et reliés par une de leurs bases. Le diamètre extérieur d₁ est choisi de sorte à être sensiblement égal au diamètre intérieur du collet 22 de l'embase 10. Le diamètre intérieur d₂ est quant à lui choisi de sorte à être sensiblement égal au diamètre extérieur du collet 22. Les longueurs du tube 50a et du tube 50b sont choisies en fonction de l'amplitude du déplacement souhaitée pour le applicateur 12 lorsque le bouton 18 est actionné.

Le tube 50a comporte trois saignées 52a s'étendant longitudinalement dans la face extérieure du premier tube 50a. Les saignées 52a sont dimensionnées et positionnées de sorte à coopérer avec les glissières 23 du collet 22 de l'embase 10 lorsque le support 50 est monté dans le collet 22. Le tube 50a comporte à son extrémité libre une collerette intérieure 54a coopérant avec une saignée circulaire pratiquée dans le tampon 14 de sorte à solidariser ce dernier au support 50. La face externe du tube 50b comporte un filet de vis 56b en saillie.

En variante, le tube 50b peut avoir deux filets « emboités » l'un dans l'autre pour fiabiliser le vissage et répartir les efforts de serrage.

Le tampon 14 est réalisé dans un matériau transparent. La forme d'appui en calotte sphérique du tampon 14 est choisie de sorte à maximiser le confort du patient tout en réduisant le risque de blocage du retour veineux sur les autres veines situées à proximité.

En variante, la forme d'appui du tampon peut être différente d'une calotte sphérique, notamment oblongue et en particulier semi-cylindrique comme représenté aux Figures 3c et 3d, afin de suivre la gouttière du poignet où se situe l'artère radiale.

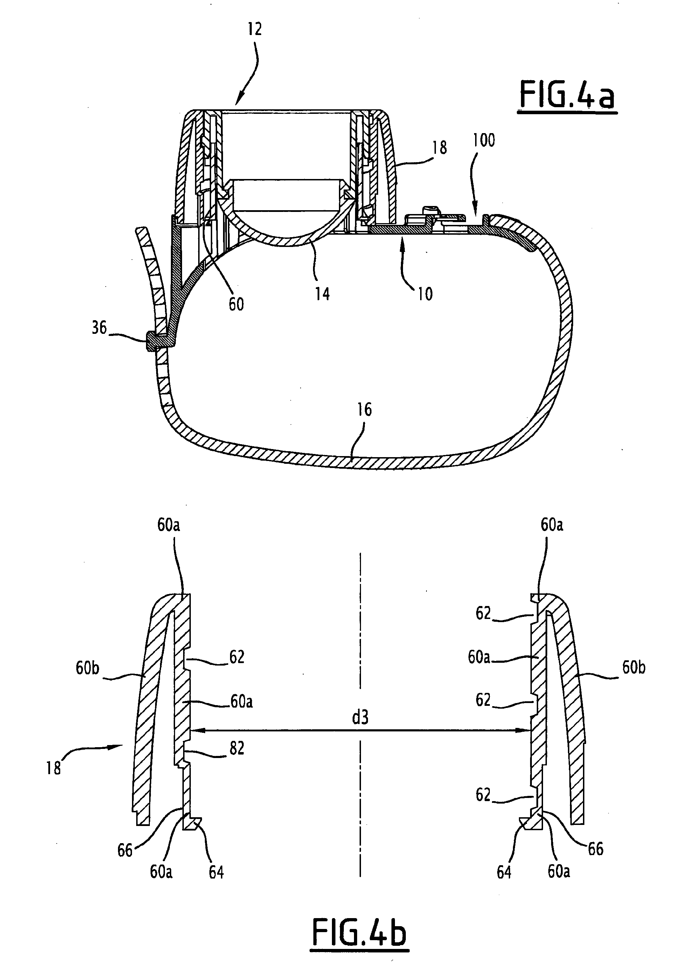

Comme illustré sur les figures 4a et 4b, le bouton 18 est formé d'un tube intérieur cylindrique 60a muni extérieurement d'un organe de préhension 60b sensiblement conique permettant de faciliter la manipulation du bouton 14 par un opérateur. Le tube cylindrique 60a a un diamètre interne d₃ sensiblement égal au diamètre extérieur du tube extérieur 50b du support 50 du applicateur 12. La face interne du tube cylindrique 60a comporte un pas de vis creux 62 apte à coopérer avec le ou chaque filet de vis 56b de l'applicateur 12. La paroi intérieure cylindrique du bouton 18 comporte encore, à sa base, une collerette intérieure 64 saillante dont les dimensions et la disposition sont choisies de sorte à coopérer, une fois le bouton 18 monté sur l'embase 10, avec les clips d'assemblage 26 de celle-ci de sorte à bloquer la translation du bouton 18 suivant son axe longitudinal mais à laisser le bouton 18 libre en rotation autour du même axe. Des crans 66 sont disposés régulièrement sur toute la face externe du tube cylindrique 60a de sorte qu'ils soient aptes à coopérer avec les godrons de cliquet 32 des deux nervures 30 lorsque le bouton 18 est monté sur l'embase 10, pour former des moyens d'encliquetage permettant de maintenir le bouton en une pluralité de positions angulaires stables prédéterminées. Si on le souhaite, pour différencier l'effort à exercer sur le bouton suivant les deux directions, les crans peuvent être asymétriques.

Pour faciliter la réalisation du bouton, la partie inférieure du bouton, comportant la collerette 64 et les crans 66, pourrait être une autre pièce assemblée (collée ou soudée par exemple) sur le reste du bouton.

Une fois assemblés, l'applicateur 12, le bouton 18 et l'embase 10 sont ainsi reliés par un système vis-écrou pour que la rotation du bouton entraîne la translation du tampon. Les filets de vis 56b, 62 du système vis-écrou sont situés entièrement à l'extérieur du diamètre du tampon transparent 14. Par conséquent, la vue à travers le tampon transparent 14 sur la zone d'introduction n'est pas occultée.

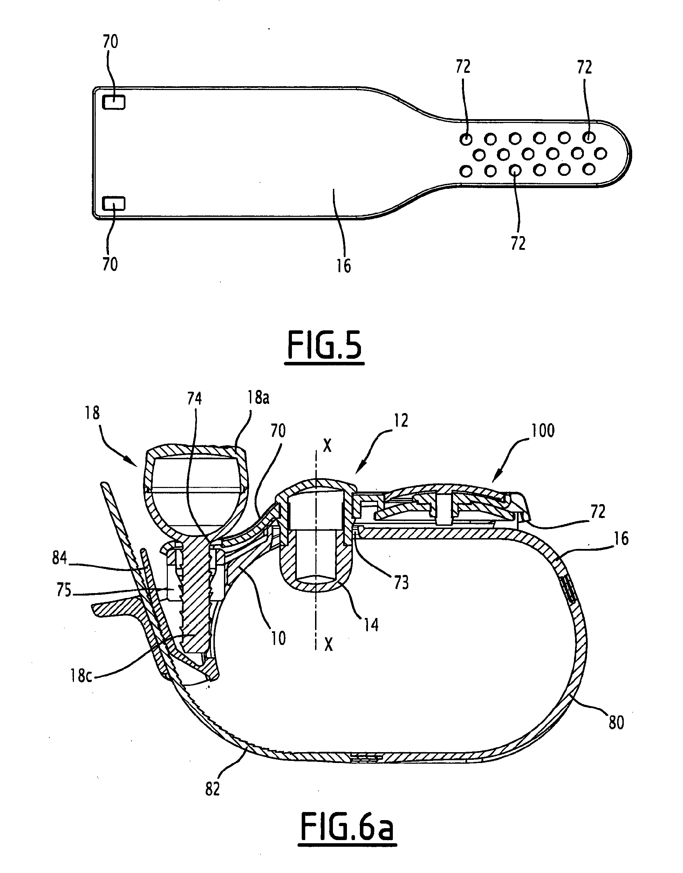

Comme représenté à la figure 5, le bracelet 16 comporte à l'une de ses extrémités deux perforations 70 dont la forme et la disposition permettent, en coopération avec les crochets 38 de l'embase 10, de solidariser ladite extrémité à l'embase. L'autre extrémité du bracelet 16 comporte une pluralité de trous débouchants 72, chacun pouvant coopérer avec le plot de fixation 36 pour solidariser ladite extrémité à l'embase 10, le choix du trou débouchant permettant de régler le serrage du bracelet 16 autour du poignet du patient.

En variante, le bouton 18 pourrait être directement vissé dans un trou de l'embase 10, avec l'extrémité proximale de l'applicateur 12 solidaire au moins en translation du bouton.

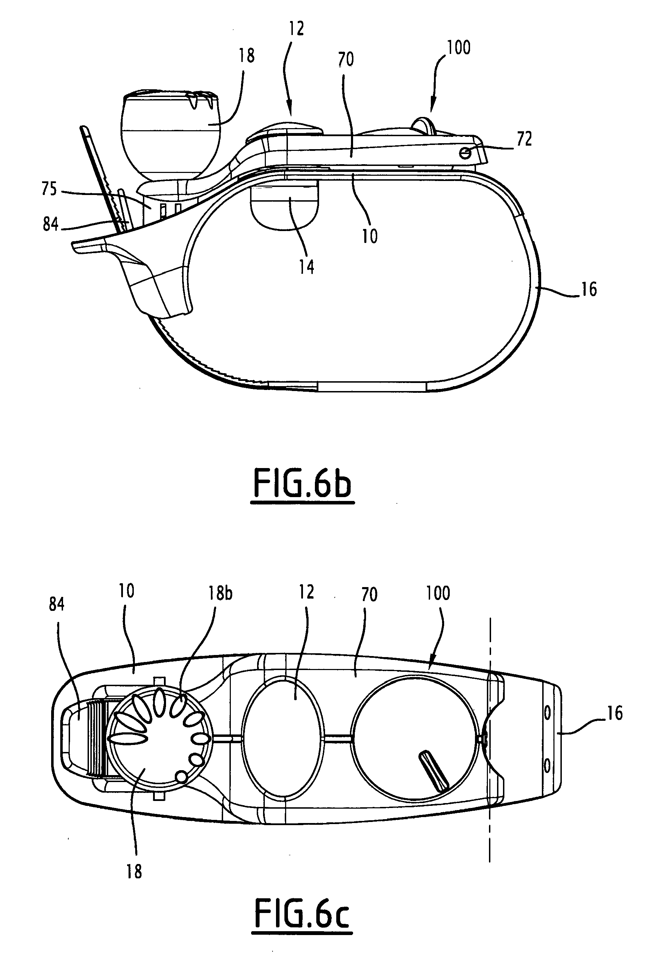

Dans un deuxième mode de réalisation du dispositif hémostatique de compression selon l'invention, représenté aux figures 6a, 6b et 6c, l'extrémité proximale de l'applicateur 12, entièrement transparent, est solidaire d'une platine 70 dont une extrémité est reliée à l'embase 10 par une charnière 72. L'applicateur d'axe X-X, traverse librement un orifice 73 de l'embase. L'autre extrémité de la platine 70 présente un trou 74 parallèle à l'axe X-X.

Le bouton 18 comporte une partie supérieure 18a formant la tête du bouton 18, sur laquelle des repères angulaires 18b sont disposés. Le bouton 18 comporte une partie inférieure 18c formant une tige filetée traversant le trou 74 et vissée dans un écrou 75 solidaire de la partie 10b de l'embase 10.

Avantageusement, l'écrou 75 est conçu de sorte à pouvoir se déformer suffisamment pour permettre la translation du bouton selon son axe longitudinal lorsqu'une pression selon cet axe est exercée sur la tête 18a par un utilisateur, permettant ainsi de déplacer plus rapidement le bouton 18 que par vissage. Pour cela, le filet de l'écrou 75 peut comporter des lames souples et être réalisé dans un matériau offrant une élasticité suffisante.

Le bracelet 16 comporte un bracelet souple 80 dont l'une des extrémités est solidaire d'une extrémité de l'embase 10 et prolonge cette dernière, l'autre extrémité comportant une zone crantée 82. La zone crantée est apte à coopérer avec un levier de verrouillage/déverrouillage 84 prévu à l'extrémité proximale de l'embase munie de l'écrou 75. Le levier 84 tend à prendre élastiquement sa position de verrouillage.

Avantageusement, le tampon 14 fait saillie par rapport à l'embase 10 vers la zone d'introduction quelle que soit sa position définie par le bouton de réglage. Ceci garantit dans tous les cas l'obtention d'une pression sur la zone d'introduction lors de la pose du dispositif.

Avantageusement, le dispositif selon l'invention peut comporter des moyens de mémorisation des heures de pose et de retrait, permettant à la personne qui manipule le dispositif de mémoriser l'heure à laquelle le dispositif a été posé sur le patient, ainsi que l'heure à laquelle le dispositif doit être retiré ou a été effectivement retiré. Les moyens de mémorisation des heures de pose et de retrait peuvent avantageusement être disposés sur l'embase 10.

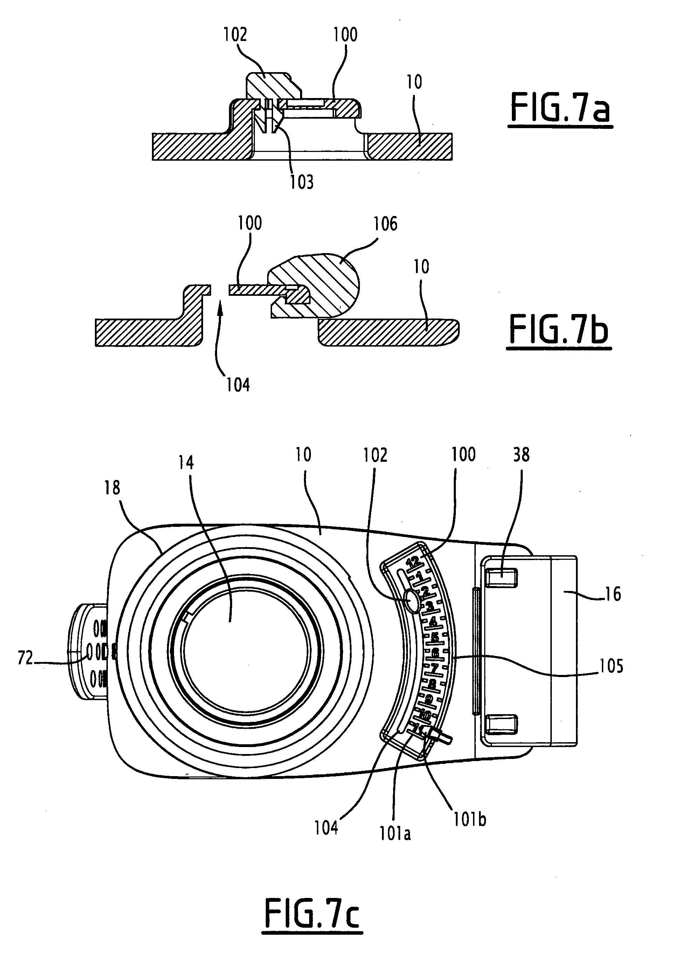

Dans un premier mode de réalisation illustré sur les figures 7a, 7b et 7c, les moyens de mémorisation des heures de pose et de retrait comportent une règle arquée 100, centrée sur l'axe X-X, sur laquelle figurent des graduations 101 a, 101 b. A chaque graduation correspond un marquage de l'heure correspondante. Typiquement, le marquage s'étend de 12 à 11, représentant une échelle horaire allant de 12 heures à 11 heures comme représenté à la figure 7c. La règle 100 comporte une première glissière crantée intérieure 104 de guidage. Un pion 102 muni de clips de montage 103 est monté dans la glissière 104. Le pion 102 peut se déplacer à frottement dans la glissière 104 et être maintenu à une pluralité de positions fixes correspondant chacune à un des marquages de la graduation intérieure 101 a. La règle 100 comporte une deuxième glissière de guidage extérieure crantée 105 sur laquelle coulisse un pointeur 106. Le pointeur 106 peut se déplacer à frottement dans la deuxième glissière crantée et être positionné à une pluralité de positions fixes correspondant chacune à un des marquages de la graduation extérieure 101 b. Des crans prévus dans les deux glissières peuvent, en variante, définir des positions prédéterminées stables du pion 102 et du pointeur 106.

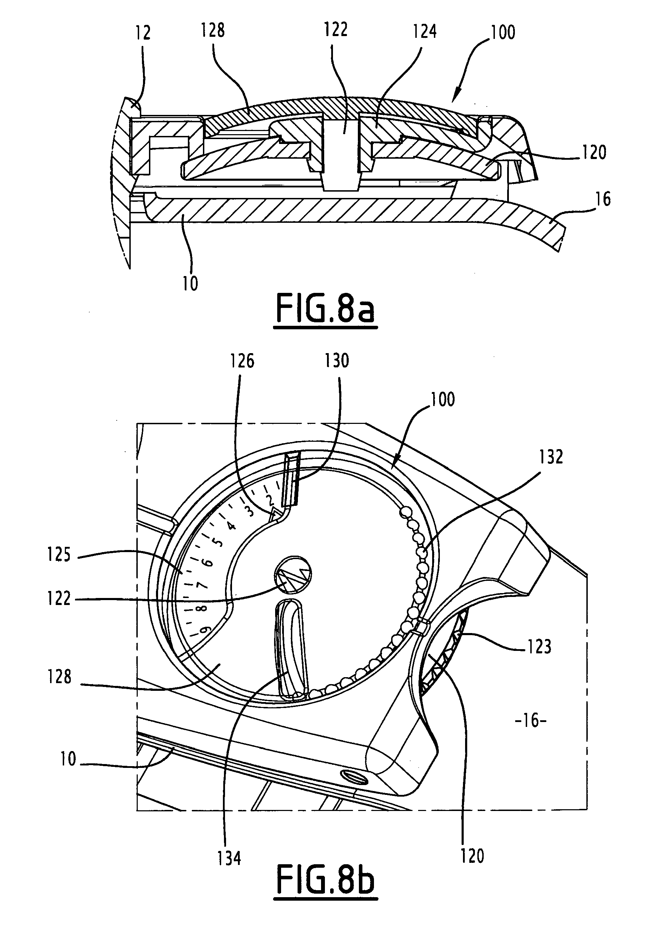

Dans un deuxième mode de réalisation illustré sur les figures 8a et 8b, les moyens 100 de mémorisation des heures de pose et de retrait comportent un cadran 120 monté rotatif autour d'un axe 122. Sur le cadran 120 sont marqués des repères horaires allant par exemple de 1 heure à 24 heures. Une partie périphérique moletée 123 du cadran est accessible à l'utilisateur de sorte que ce dernier soit en mesure de pouvoir entraîner la rotation du cadran 120. Le cadran 120 est partiellement masqué par un disque opaque fixe 124 couvrant le cadran 120. Le disque opaque 124 comporte en outre un index fixe 126 disposé de sorte à permettre le pointage d'un repère horaire du cadran 120 non couvert par le disque opaque 124. La partie du cadran 120 laissée visible par le disque opaque 124 correspond à une zone en secteur circulaire 125 dont les dimensions permettent de visualiser un ensemble de repères horaires du cadran 120 correspondant à une plage horaire de 8 heures à partir de l'heure pointée par l'index fixe 126. Autour de l'axe 122 est également monté à rotation un disque transparent 128 recouvrant le disque opaque 124. Le disque transparent 128 est muni d'un pointeur 130 disposé de sorte à ce qu'un utilisateur puisse identifier un marquage horaire du cadran 120 non couvert par le disque opaque 124. Des crans d'indexation 132 sont disposés sur le disque opaque 124 et sur le disque transparent 128 de manière à indexer la rotation du disque transparent 128 sur une pluralité de positions angulaires prédéterminées. Pour faciliter la mise en rotation du disque transparent 128 par un utilisateur, un renflement 134 est disposé en surface du disque transparent 128.

L'utilisateur peut ainsi mémoriser l'heure de pose et de mise en oeuvre du dispositif sur le patient en faisant tourner le cadran 120 jusqu'à ce que le marquage du cadran 120 correspondant à ladite heure de pose soit pointé par l'index fixe 126. L'utilisateur peut indiquer l'heure de retrait du dispositif en faisant tourner le disque transparent 128 jusqu'à ce que le pointeur 130 désigne le marquage du cadran 120 correspondant à ladite heure de retrait.

En variante, dans chaque mode de réalisation, l'une et/ou l'autre des indications horaires peut repérer une heure de modification, effectuée ou à venir, de la pression exercée par le dispositif.

Une combinaison des deux modes de réalisation des moyens de mémorisation illustrés sur les Figures 7a à 7c et 8a-8b peut être envisagée.

On peut également; en variante, réaliser un dispositif ne comportant qu'une seule graduation horaire.

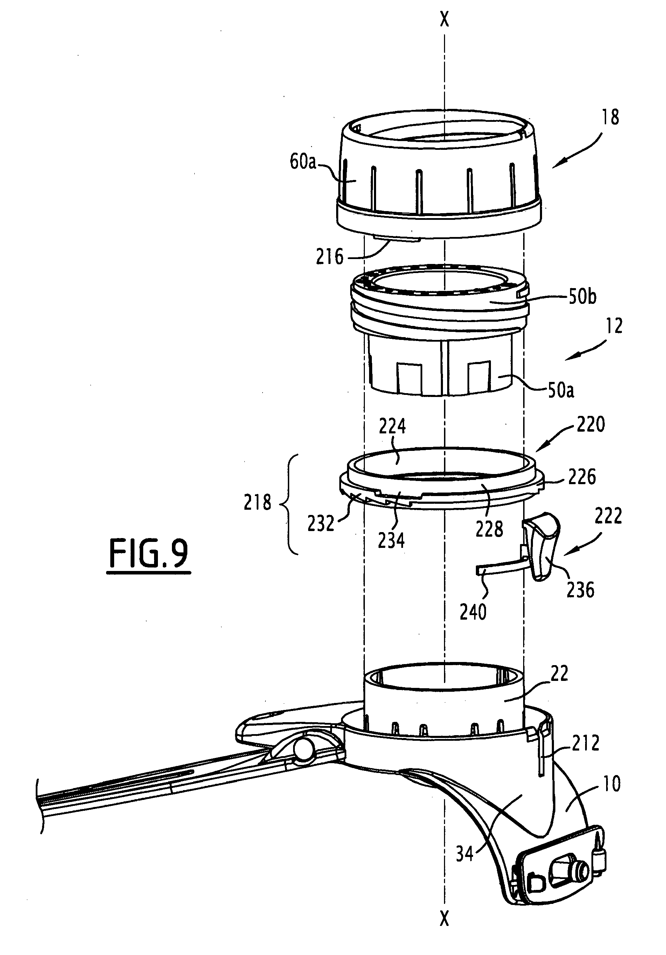

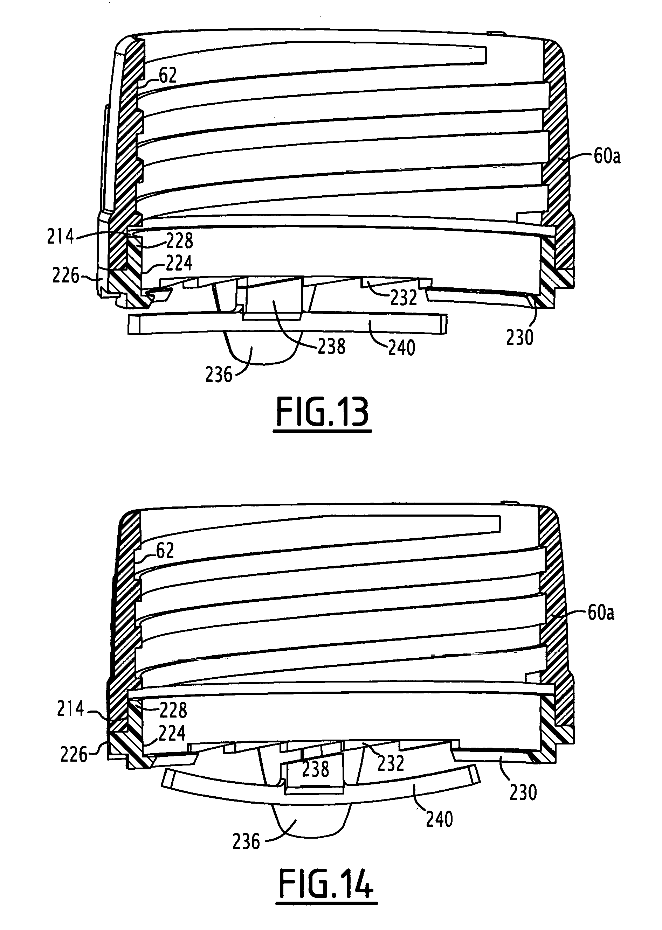

Un troisième mode de réalisation du dispositif hémostatique est représenté sur les figures 9 à 14. Ce dispositif diffère du premier mode de réalisation uniquement par ce qui suit :
- l'embase 10 ne comporte pas les supports 30 avec les saillies de cliquet 32, mais deux appuis verticaux extérieurs 200 et un appui vertical intermédiaire 210, dépassant de l'embase 10 vers le bouton 18. Le support 210 se trouve dans le plan vertical de symétrie P du dispositif, et les supports 200 à environ 30° de part et d'autre de ce plan.
- l'extension 34 s'élève au-dessus du plan de la partie plane 10a de l'embase 10, est circulaire et présente une fente verticale 212 dans le plan P ;
- le bouton 18 ne comporte pas d'organe de préhension 60b, mais seulement le tube cylindrique intérieur 60a, ce tube 60a étant également un organe de préhension ; et
- le tube cylindrique 60a ne présente ni les crans 66, ni la collerette intérieure 64 saillante, mais comporte dans sa partie orientée vers l'embase 10 (figures 13 et 14) un évidement intérieur 214 et au moins une languette 216 qui dépasse verticalement du bouton 18 vers l'embase 10 (figure 9).

Par rapport au premier mode de réalisation, le troisième mode de réalisation du dispositif hémostatique comporte en outre un système de verrouillage 218.

Le système de verrouillage 218 comprend une bague 220 et un loquet anti-retour 222. La bague 220 est placée dans l'espace annulaire situé entre le collet 22 et l'extension 34.

La bague 220 comprend un anneau cylindrique intérieur 224 et une couronne dentée 226 extérieure. La bague 220 est d'un seul tenant. La bague 220 est réalisée en un copolymère, par exemple en ABS. L'anneau 224 et la couronne dentée 226 s'étendent concentriquement autour de l'axe longitudinal X-X. La couronne dentée 226 entoure l'anneau 224. Ce dernier dépasse verticalement de la couronne dentée 226 vers le bouton 18 en formantm un collet 228. L'anneau 224 présente dans sa partie orientée vers l'embase 10 une collerette intérieure saillante 230 dont la fonction est analogue à celle de la collerette 64 correspondante du bouton 18 du premier mode de réalisation : en coopérant avec les clips 26 de l'embase, la collerette 230 bloque la translation de la bague 220 suivant l'axe longitudinal X-X mais laisse la bague 220 libre en rotation autour du même axe.

La couronne 226 présente sur sa tranche d'extrémité orientée vers l'embase 10 des dents de scie 232 orientées verticalement. Les dents 232 sont réparties régulièrement sur la totalité de la périphérie de la couronne dentée 226. En variante, les dents 232 sont réparties régulièrement sur une partie correspondant à 180° ou 270° de la périphérie de la couronne 226. Dans sa partie orientée à l'opposé de l'embase, la couronne 226 présente au moins une encoche 234 (figure 9).

Le collet 228 de la bague 220 a un diamètre extérieur adapté pour un serrage par friction dans l'évidement 214 du tube cylindrique 60a du bouton 18 (figures 13 et 14). La ou chaque encoche 234 de la bague 220 est adaptée pour coopérer avec la ou chaque languette 216 du bouton 18. Ainsi, la bague 220 est solidaire en rotation du bouton 18 autour de l'axe X-X

L'applicateur 12 et son tampon transparent 14 sont sensiblement indentiques à ceux du premier mode de réalisation.

En variante, la bague 220 et le bouton 18 sont reliés l'un à l'autre par collage ou soudage par ultrasons.

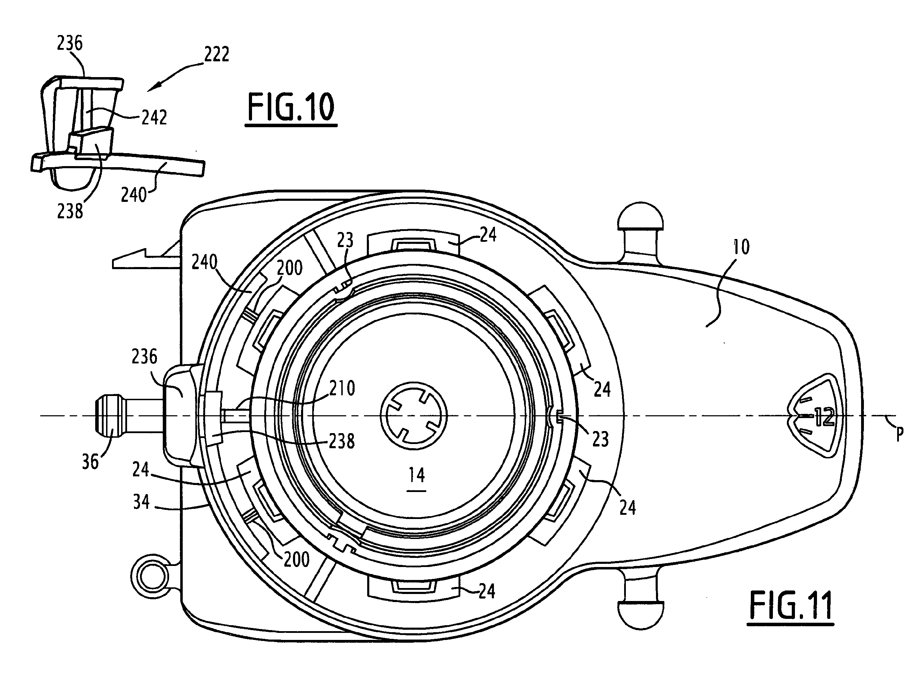

Le loquet anti-retour 222 comprend un poussoir de commande 236, un cliquet de verrouillage 238, un bras arqué 240 et une nervure verticale 242 (figure10). Le loquet 222 est d'un seul tenantet est réalisé en un polymère, tel que du POM.

Le poussoir de commande 236 est relié au cliquet 238 au moyen de la nervure verticale 242. Il est adapté pour déplacer le cliquet 238 parallèlement à l'axe X-X entre une position de blocage et une position de déblocage. Le cliquet 238 présente dans sa partie orientée à l'opposé de l'embase une forme complémentaire à celle de l'une des dents 232 de la couronne dentée 226, de façon à pouvoir coopérer avec chacune des dents 232. La partie orientée vers l'embase 10 du cliquet 238 est reliée sensiblement à la partie centrale du bras arqué 240.

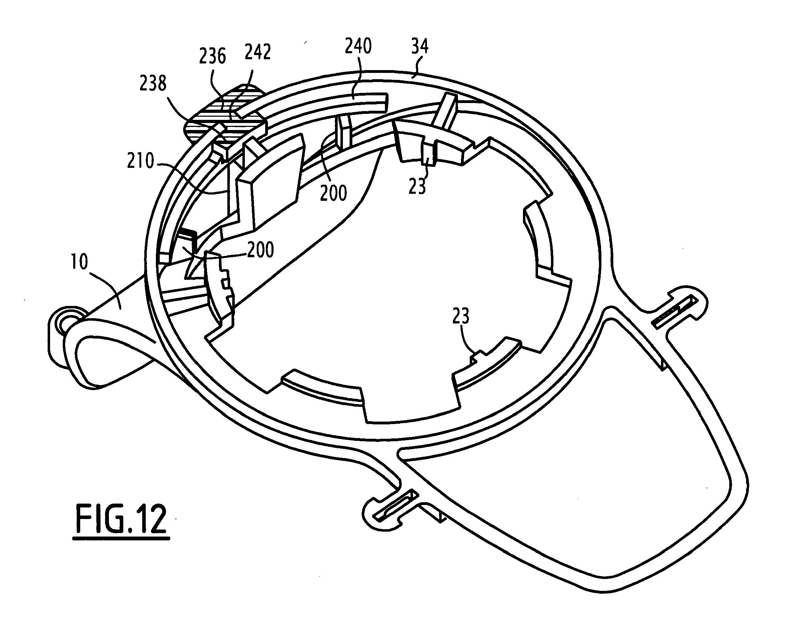

Dans la position de blocage, le bras arqué 240 s'étend sensiblement dans un plan perpendiculaire à l'axe X-X de part et d'autre du cliquet 238 (figure 12). Le bras arqué 240 décrit un arc de cercle ayant un diamètre égal ou supérieure au diamètre extérieur d₁ du tube intérieur 50a de l'applicateur 12.

La fente verticale 212 présente dans l'extension 34 de l'embase 10 s'élève au-dessus du plan de la partie plane 10a de l'embase et est ouverte à l'opposé de l'embase (figure 9). La fente 212 a une largeur et une longueur adaptées pour recevoir la nervure verticale 242 du loquet 222 de façon telle que le poussoir 236 vient en appui glissant contre la face extérieure de l'extension 34 et que le cliquet 238 et le bras arqué 240 viennent en appui glissant contre la face intérieure de l'extension 34 (figure 11). Par conséquent, le poussoir 236 est accessible par un utilisateur, tandis que le cliquet 238 et le bras arqué 240 ne le sont pas.

Par ailleurs, (figure 11), les parties d'extrémité du bras 240 se trouvent à l'aplomb des appuis 200 de l'embase.

Comme représenté sur la figure 11, le loquet anti-retour 222 se trouve entièrement à l'extérieur du diamètre du tampon transparent 14.

La figure 12 montre les deux appuis verticaux extérieurs 200 et l'appui vertical intermédiaire 210, dépassant de l'embase vers le bouton 18. Le appuis 210 dépasse de l'embase 10 jusqu'à une hauteur moins importante que les appuis 200. Les hauteurs des appuis 200, 210 sont telles que le bras arqué 240 est en appui uniquement contre les appuis extérieurs 200 lorsque le cliquet 238 est en position de blocage, et que le bras 240 est fléchi en permanence, c'est-à-dire lorsque le cliquet 238 est en position de blocage ou de déblocage.

Lorsque le cliquet 238 est en position de blocage (figure 12), la partie du cliquet orientée à l'opposé de l'embase 10 est en appui contre l'une des dents 232 de la couronne dentée 226. L'inclinaison des dents 232 permet la rotation unidirectionnelle autour de l'axe X-X de la bague 220 et donc du bouton 18 lorsque le cliquet 238 est dans cette position de blocage. Dans cet d'exemple, la rotation du bouton 18 est bloquée dans le sens de rotation entraînant l'applicateur 12 et le tampon 14 à l'opposé de la zone d'introduction, alors que la rotation dans le sens opposé, entraînant l'applicateur 12 et le tampon 14 vers la zone d'introduction, n'est pas bloquée. Le bras arqué 240 n'est en appui que sur les appuis 200.

Pour permettre la rotation du bouton 18 dans le premier sens précité, c'est-à-dire pour réduire la pression exercée par le tampon 14, l'utilisateur presse le poussoir 236, à l'encontre de la force élastique exercée par le bras arqué 240. Ceci amène le cliquet 238 en position de déblocage (figure 14), dans laquelle il n'est pas en contact avec les dents 232 de la couronne dentée 226. Le bras arqué 240 est alors incurvé verticalement et vient en appui contre et l'appui intermédiaire 210. Lorsque le cliquet 238 est en position de déblocage, la bague 220 et donc le bouton 18 sont libres de tourner dans les deux sens autour de l'axe X-X. Dans cette position, il est donc possible d'entraîner l'applicateur 12 et le tampon 14 à l'opposé de la zone d'introduction afin de réduire la pression exercée sur cette zone.

Afin de réduire la pression du tampon 14 exercée sur la zone d'introduction, il est impératif d'amener le loquet anti-retour 222 dans la position de déblocage et de tourner le bouton 18 dans le sens correspondant. Ces deux manoeuvres sont difficiles ou impossibles de réaliser avec une seule main, par exemple par un patient portant le dispositif hémostatique autour de l'un de ses poignets.

Le système de verrouillage 218 est disposé entièrement à l'extérieur du diamètre du tampon 14 de sorte à ne pas occulter la vue à travers le tampon transparent 14 sur la zone d'introduction. Ceci permet à un utilisateur de positionner le dispositif hémostatique selon l'invention de façon optimale sur la zone d'introduction d'un patient.

## Revendications

1. Dispositif hémostatique par compression apte à stopper les saignements provoqués par le retrait d'un introducteur piqué dans une zone d'introduction d'un patient, notamment pour réaliser l'hémostase de l'artère radiale, le dispositif étant du type comprenant :
• une embase (10) ;
• des moyens de maintien (16) aptes à fixer temporairement l'embase (10) au patient;
• un applicateur (12) porté par l'embase et muni d'un tampon (14) ; et
• des moyens de réglage (18) aptes à entraîner l'applicateur (12) vers la zone d'introduction de sorte que l'applicateur (12) se déplace jusqu'à une position où le tampon (14) exerce une pression sur la zone d'introduction,
l'embase (10) comportant un trou de visée (20) débouchant maintenu par les moyens de maintien (16) en regard de la zone d'introduction, le tampon (14) étant transparent, et les moyens de réglage (18) étant situés entièrement à l'extérieur du diamètre du tampon transparent (14); **caractérisé en ce que** le tampon se déplace par rapport à l'embase, en étant mobile dans le trou de visée.

2. Dispositif selon la revendication 1, dans lequel les moyens de réglage comportent un bouton (18) monté rotatif sur l'embase (10) autour de l'axe longitudinal (X-X) du trou de visée (20), l'applicateur (12), le bouton (18) et l'embase (10) étant reliés par un système vis-écrou pour que la rotation du bouton (18) entraîne la translation de l'applicateur (12) le long dudit axe longitudinal (X-X) ;
et dans lequel le système vis-écrou comprend des filets de vis (56b, 62) de l'applicateur (12) et du bouton (18) coopérant ensemble, les filets de vis (56b, 62) étant situés entièrement à l'extérieur du diamètre du tampon transparent (14).

3. Dispositif selon la revendication 1 ou 2, dans lequel le tampon (14) fait saillie par rapport à l'embase (10) vers la zone d'introduction quelle que soit sa position définie par les moyens de réglage (18).

4. Dispositif selon la revendication 2 ou 3, dans lequel les filets de vis (56b, 62) sont prévus sur une face externe de l'applicateur (12) et sur une face interne du bouton (18).

5. Dispositif selon l'une quelconque des revendications 1 à 4, dans lequel l'embase (10) et le bouton (18) comportent des moyens d'encliquetage coopérants (30, 32, 66) permettant de maintenir le bouton en une pluralité de positions angulaires stables prédéterminées.

6. Dispositif selon l'une quelconque des revendications 1 à 5, comportant un système de verrouillage (218) adapté pour bloquer la rotation du bouton (18) dans l'un des deux sens de rotation du bouton (18).

7. Dispositif selon la revendication 6, dans lequel le système de verrouillage (218) est un système de verrouillage unidirectionnel adapté pour bloquer la rotation du bouton (18) dans le sens de rotation entraînant la translation de l'applicateur (12) à l'opposé de la zone d'introduction.

8. Dispositif selon la revendication 6 ou 7, dans lequel le système de verrouillage (218) comprend un organe de verrouillage (238) mobile entre une position de blocage et une position de déblocage.

9. Dispositif selon la revendication 8, dans lequel l'organe de verrouillage (238) est mobile parallèlement audit axe longitudinal (X-X).

10. Dispositif selon la revendication 9, dans lequel l'organe de verrouillage (238) est solidaire d'un bras arqué (240) qui prend appui sur l'embase pour assurer un rappel élastique de l'organe de verrouillage (238) vers sa position de blocage.

11. Dispositif selon l'une quelconque des revendications 6 à 10, dans lequel le système de verrouillage (218) est situé entièrement à l'extérieur du diamètre du tampon transparent (14).

12. Dispositif hémostatique selon l'une quelconque des revendications précédentes, dont les moyens de maintien (16) comportent un bracelet solidaire de l'embase (10).

13. Dispositif selon l'une quelconque des revendications précédentes, comportant des moyens (100) permettant de mémoriser l'heure à laquelle ledit dispositif a été posé sur le patient ou la durée écoulée depuis cet instant.

14. Dispositif selon l'une quelconque des revendications précédentes, comportant des moyens (100) permettant de mémoriser l'heure à laquelle ledit dispositif doit être retiré du patient ou la durée restante jusqu'à cet instant.

15. Dispositif selon l'une quelconque des revendications 13 ou 14, comportant en outre des moyens (100) permettant de mémoriser l'heure à laquelle la pression exercée par le dispositif a été ou doit être modifiée ou la durée écoulée depuis cet instant.

## Patentansprüche

1. Blutstillende Vorrichtung via Kompression, die in der Lage ist Blutungen zu stoppen, die hervorgerufen sind durch das Rückziehen einer Einführvorrichtung, die in eine Einführzone eines Patienten eingestochen ist, insbesondere zum Realisieren der Blutstillung der Radialarterie, wobei die Vorrichtung des Typs ist, der aufweist:
• einen Sockel (10),
• Haltemittel (16), die eingerichtet sind, um temporär den Sockel (10) am Patienten zu fixieren,
• einen Applikator (12), der von dem Sockel getragen ist und der mit einem Stopfen (14) versehen ist, und
• Einstellmittel (18), die eingerichtet sind, um den Applikator (12) zu der Einführzone hin anzutreiben derart, dass der Applikator (12) sich bis in eine Position verlagert, wo der Stopfen (14) einen Druck auf die Einführzone ausübt,
wobei der Sockel (10) ein ausmündendes Sehloch (20) aufweist, das von den Haltemitteln (16) gegenüber der Einführzone gehalten wird, wobei der Stopfen (14) transparent ist, und wobei die Einstellmittel (18) gänzlich am Äußeren des Durchmessers des transparenten Stopfen (14) angeordnet sind, **dadurch gekennzeichnet, dass** der Stopfen sich bezüglich des Sockels verlagert indem er in dem Sehloch bewegbar ist.

2. Vorrichtung gemäß Anspruch 1, wobei die Einstellmittel einen Drehknopf (18) aufweisen, der um die Längsachse (X-X) des Sehlochs (20) drehbar an dem Sockel (10) montiert ist, wobei der Applikator (12), der Drehknopf (18) und der Sockel (10) durch ein Schraube-Mutter-System realisiert sind, sodass die Drehung des Drehknopfs (18) die Translation des Applikators (12) entlang der Längsachse (X-X) antreibt, und wobei das Schraube-Mutter-System Schraubengewinde (56b, 62) des Applikators (12) und des Drehknopfs (18) aufweist, die zusammenwirken, wobei die Schraubengewinde (56b, 62) gänzlich am Äußeren des Durchmessers des transparenten Stopfens (14) angeordnet sind.

3. Vorrichtung gemäß Anspruch 1 oder 2, wobei der Stopfen (14) bezüglich des Sockels (10) zu der Einführzone hin vorsteht wie auch immer seine Position ist, die von den Einstellmitteln (18) definiert ist.

4. Vorrichtung gemäß Anspruch 2 oder 3, wobei die Schraubengewinde (56b, 62) an der Außenfläche des Applikators (12) und an der Innenfläche des Drehknopfs (18) ausgebildet sind.

5. Vorrichtung gemäß einem der Ansprüche 1 bis 4, wobei der Sockel (10) und der Stopfen (18) zusammenwirkende Rastmittel (30, 32, 66) aufweisen, die ermöglichen, den Drehknopf in einer Mehrzahl von stabilen, vorbestimmten Winkelpositionen zu halten.

6. Vorrichtung gemäß einem der Ansprüche 1 bis 5, aufweisend ein Verriegelungssystem (218), das eingerichtet ist zum Blockieren der Drehung des Drehknopfs (18) in einer der beiden Drehrichtungen des Drehknopfs (18).

7. Vorrichtung gemäß Anspruch 6, wobei das Verriegelungssystem (218) ein unidirektionales Verriegelungssystem ist, das eingerichtet ist zum Blockieren der Drehung des Drehknopfs (18) in jener Drehrichtung, die die Translation des Applikators (12) entgegen der Einführzone antreibt.

8. Vorrichtung gemäß Anspruch 6 oder 7, wobei das Verriegelungssystem (218) ein Verriegelungsorgan (238) aufweist, das zwischen einer Blockier-Position und einer Freigabe-Position bewegbar ist.

9. Vorrichtung gemäß Anspruch 8, wobei das Verriegelungsorgan (238) parallel zu der Längsachse (X-X) bewegbar ist.

10. Vorrichtung gemäß Anspruch 9, wobei das Verriegelungsorgan (238) mit einem gekrümmten Arm (240) fest verbunden ist, der sich an dem Sockel abstützt zum Gewährleisten einer elastischen Rückstellung des Verriegelungsorgans (238) zu dessen Blockier-Position hin.

11. Vorrichtung gemäß einem der Ansprüche 6 bis 10, wobei das Verriegelungssystem (218) gänzlich am Äußeren des Durchmessers des transparenten Stopfens (14) angeordnet ist.

12. Blutstillende Vorrichtung gemäß einem der vorhergehenden Ansprüche, deren Haltemittel (16) ein Armband aufweisen, das mit dem Sockel (10) fest verbunden ist.

13. Vorrichtung gemäß einem der vorhergehenden Ansprüche, aufweisend Mittel (100), die ermöglichen zu speichern den Zeitpunkt, zu welchem die Vorrichtung an den Patienten angelegt worden ist, oder die Zeitdauer, die seit diesem Augenblick verstrichen ist.

14. Vorrichtung gemäß einem der vorhergehenden Ansprüche, aufweisend Mittel (100), die ermöglichen zu speichern den Zeitpunkt, zu welchem die Vorrichtung von dem Patienten abzunehmen ist, oder die Zeitdauer, die noch bis zu diesem Augenblick verbleibt.

15. Vorrichtung gemäß einem der Ansprüche 13 oder 14, ferner aufweisend Mittel (100), die ermöglichen zu speichern den Zeitpunkt, zu welchem der Druck, der von der Vorrichtung ausgeübt wird, modifiziert worden ist oder modifiziert werden muss, oder die Zeitdauer, die seit diesem Augenblick verstrichen ist.

## Claims

1. A compressive hemostatic device capable of stopping the bleeding caused by the withdrawal of an injector stuck into an injection area of a patient, in particular to perform the hemostasis of the radial artery, the device being of the type comprising:
• a base (10) ;
• supporting means (16) capable of temporarily attaching the base (10) to the patient;
• an applicator (12) supported by the base and provided with a pad (14); and
• adjustment means (18) capable of moving the applicator (12) toward the injection area so that the applicator (12) moves to a position where the pad (14) exerts pressure on the injection area,
the base (10) comprising a through sighting hole (20) supported by the supporting means (16) opposite the injection area, the pad (14) being transparent, and the adjustment means (18) being situated completely outside the diameter of the transparent pad (14), **characterized in that** the pad moves relative to the base and is movable in the sighting hole.

2. The device according to claim 1, wherein the adjusting means comprise a button (18) rotatably mounted on the base (10) around the longitudinal axis (X-X) of the sighting hole (20), the applicator (12), the button (18) and the base (10) being connected by a screw-and-nut system so that the rotation of the button (18) causes the translation of the applicator (12) along said longitudinal axis (X-X);
and wherein the screw-and-nut system comprises screw threads (56b, 62) of the applicator (12) and the button (18) cooperating together, the screw threads (56b, 62) being situated completely outside the diameter of the transparent pad (14).

3. The device according to claim 1 or 2, wherein the pad (14) protrudes relative to the base (10) toward the injection area irrespective of its position defined by the adjusting means (18).

4. The device according to claim 2 or 3, wherein the screw threads (56b, 62) are provided on an outer face of the applicator (12) and on an inner face of the button (18).

5. The device according to any one of claims 1 to 4, wherein the base (10) and the button (18) comprise cooperating stop means (30, 32, 66) making it possible to maintain the button in a plurality of predetermined stable angular positions.

6. The device according to any one of claims 1 to 5, comprising a locking system (218) adapted to block the rotation of the button (18) in one of the two directions of rotation of said button (18).

7. The device according to claim 6, wherein the locking system (218) is a unidirectional locking system adapted to block the rotation of the button (18) in the direction of rotation driving the translation of the applicator (12) opposite the injection area.

8. The device according to claim 6 or 7, wherein the locking system (218) comprises a locking member (238) able to move between a blocking position and an unblocking position.

9. The device according to claim 8, wherein the locking member (238) is movable parallel to said longitudinal axis (X-X).

10. The device according to claim 9, wherein the locking member (238) is secured to a bowed arm (240) that bears on the base to ensure an elastic return of the locking member (238) toward its blocking position.

11. The device according to any one of claims 6 to 10, wherein the locking system (218) is situated completely outside the diameter of the transparent pad (14).

12. The hemostatic device according to any one of the preceding claims, whereof the maintaining means (16) comprise a bracelet secured to the base (10).

13. The device according to any one of the preceding claims, comprising means (100) making it possible to save the time at which said device was placed on the patient or the time elapsed since that moment.

14. The device according to any one of the preceding claims, comprising means (100) making it possible to save the time at which said device must be withdrawn from the patient or the duration remaining until that moment.

15. The device according to any one of claims 13 or 14, also comprising means (100) making it possible to save the time at which the pressure exerted by the device has been or must be modified or the time elapsed since that moment.
